# EUROPÄISCHE PATENTSCHRIFT

(11) **EP 2 091 493 B1**
(45) Veröffentlichungstag und Bekanntmachung des Hinweises auf die Patenterteilung: **23.11.2011**
(21) Anmeldenummer: 07724662.7
(22) Anmeldetag: 27.04.2007
(51) Int. Cl.: A61F 13/56

(54) **ABSORBIERENDER INKONTINENZARTIKEL**
ABSORBENT INCONTINENCE ARTICLE
ARTICLE ABSORBANT POUR ÉNURÉSIE

(30) Priorität: 23.10.2006 DE 102006050971
(43) Veröffentlichungstag der Anmeldung: 26.08.2009
(73) Patentinhaber: Paul Hartmann AG, 89522 Heidenheim (DE)
(72) Erfinder: HORNUNG, Fridmann, Penalolén, Santiago (CL); KESSELMEIER, Rüdiger, 89542 Herbrechtingen (DE); GAUSE, Enno, 89522 Heidenheim (DE); BÖHMLER, Andreas, 89518 Heidenheim (DE)
(74) Vertreter: Friz, Oliver
(86) Internationale Anmeldenummer: PCT/EP2007/003733
(87) Internationale Veröffentlichungsnummer: WO 2008/049468

(56) Entgegenhaltungen:
- WO-A-2005/102241
- US-A- 3 616 114
- US-A- 3 848 594
- US-A- 4 055 182
- US-A- 4 209 016
- US-A- 5 899 895

## Beschreibung

Die Erfindung betrifft einen absorbierenden Inkontinenzartikel gemäß Oberbegriff des Anspruchs 1. Ein derartiger Artikel ist bekannt aus US-A-5,899,895.

Andere Inkontinenzartikel sind bekannt und beispielsweise in WO 2005/102241 A1 beschrieben. Die Seitenabschnitte, die mitunter auch als Ohren bezeichnet werden, werden vorzugsweise im Cut & Place-Verfahren direkt an den Hauptteil, das Chassis des Hygieneartikels angefügt. Diese Fertigungstechnologie erlaubt es, die Seitenabschnitte aus einem anderen Rohmaterial zu fertigen als den mittleren Hauptteil des Hygieneartikels. Beispielsweise können die Seitenabschnitte luftdurchlässig ausgeführt werden, wohingegen der mittlere Hauptteil im Wesentlichen feuchtigkeitsundurchlässig ausgebildet werde kann.

Die aus der Fertigungssicht effizienteste und einfachste sowie kostengünstigste Form der Seitenabschnitte ist die rechteckige Form. Sie erlaubt bei der Herstellung den Transport der die Seitenabschnitte bildenden Materialien in Form einer endlosen Flachmaterialbahn, von der dann die Seitenabschnitte quer zur Maschinenrichtung abgetrennt werden. Ein Schnittabfall ist hier praktisch nicht gegeben. Es ist jedoch durchaus denkbar, dass die Seitenabschnitte eine an sich beliebige Kontur erhalten, also insbesondere zur Längs- oder Querrichtung des Hygieneartikels geneigt oder geschwungen ausgebildet sein können. Solchenfalls entsteht jedoch ein mit Kosten verbundener Schnittabfall, jedenfalls dann, wenn die Seitenabschnitte unmittelbar im Cut & Place-Verfahren abgetrennt, angeordnet und gefügt werden.

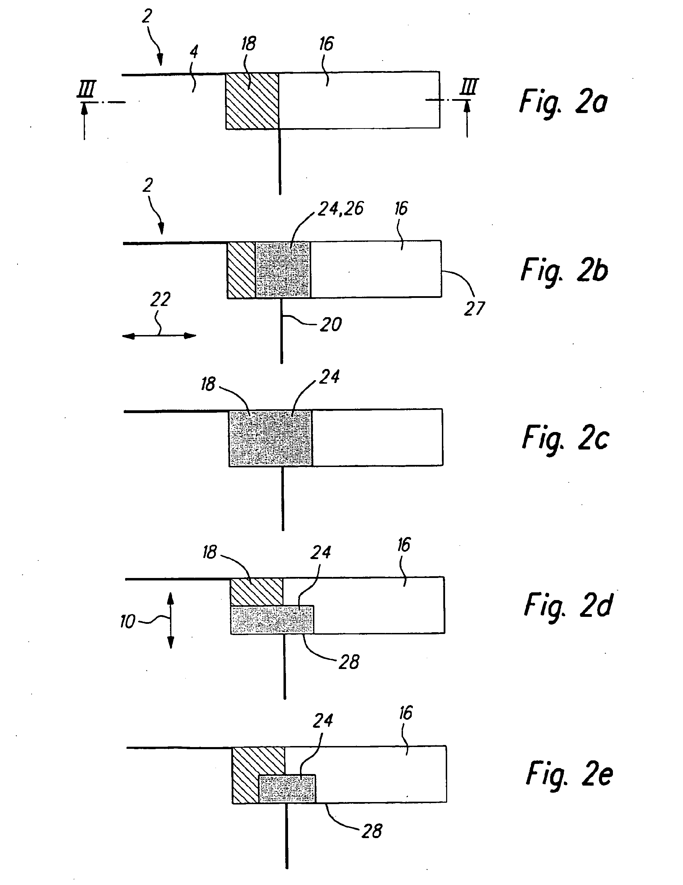

Es hat sich jedoch gezeigt, dass insbesondere bei der Ausbildung der Seitenabschnitte in der ansonsten vorteilhaften Rechteckform beim Anlegen und Tragen des Hygieneartikels mitunter das Problem besteht, dass die angefügten Seitenabschnitte im Bereich der seitlichen Längsränder des Hauptteils einreißen können. Es hat sich nämlich gezeigt, dass Anwender beim Anlegen des Hygieneartikels geneigt sind, einen zur Quer- und Längsrichtung des Hygieneartikels schrägen Zug auf die Seitenabschnitte auszuüben, was in Figur 2a mit einem schräg nach oben geneigten Pfeil angedeutet ist. Es kann solchenfalls vorkommen, dass Seitenabschnitte entlang der seitlichen Längsränder des Hauptteils einreißen, wobei der Riss ausgehend von dem dem Schrittbereich zugewandten Querrand des Seitenabschnitts ausgeht. Bislang wurde versucht; die Anfügung von derartigen Seitenabschnitten an den Hauptteil von Hygieneartikeln durch ein optimiertes Fügemuster zu verbessern, gemäß WO 2004/017882 A2 und WO 02/17843 A2.

Der vorliegenden Erfindung liegt die Aufgabe zugrunde, das vorstehend geschilderte Problem noch effektiver zu lösen, also absorbierende Inkontinenzartikel mit wenigstens zwei seitlich angestückten und angefügten Seitenabschnitten zu schaffen, bei denen das Ausreißverhalten der Seitenabschnitte signifikant verbessert ist.

Diese Aufgabe wird durch einen absorbierenden Inkontinenzartikel mit den Merkmalen des Anspruchs 1 gelöst.

Mit dem erfindungsgemäßen Vorschlag wird erstmalig nicht nur der unmittelbare Überlappungsbereich eines jeweiligen Seitenabschnitts mit dem Hauptteil optimiert, wo die Fügeverbindung zwischen Seitenabschnitt und Hauptteil vorgesehen ist, sondern es wird eine Verstärkung in einem den Längsrand des Hauptteils überfangenden Bereich vorgesehen. Die Verstärkung erstreckt sich also über den Längsrand des Hauptteils in Richtung weiterer Quererstreckung des Seitenabschnitts hinaus. Auf diese Weise wurde die Einreißfestigkeit der Seitenabschnitte in beträchtlichem Maße erhöht.

Es erweist sich als besonders vorteilhaft, dass das Verstärkungsmittel im Wesentlichen wenigstens nahezu bis zu einem dem Schrittbereich zugewandten Querrand des Seitenabschnitts erstreckt ist, wenn es also vorzugsweise kantenbündig mit dem Querrand des Seitenabschnitts abschließt oder den Querrand einschließt oder umschließt oder über den Querrand übersteht.

Das Verstärkungsmittel könnte in der Längsrichtung des Hygieneartikels, beispielsweise über die gesamte Längsausdehnung des angefügten Seitenabschnitts erstreckt sein. Es hat sich indessen gezeigt, dass dies nicht zwingend erforderlich ist, sondern dass es sich als ebenfalls vorteilhaft erweist, wenn das Verstärkungsmittel in Längsrichtung des Hygieneartikels eine geringere Abmessung aufweist als der angefügte Seitenabschnitt selbst. Infolge der beim Gebrauch auftretenden vorausgehend erörterten Krafteinwirkung auf den Seitenabschnitt und auf den Anfügungsbereich von Seitenabschnitt und Windelhauptteil ist es durchaus hinreichend, wenn das Verstärkungsmittel sich beispielsweise nur bis 80 % oder insbesondere bis 60 % und weiter insbesondere bis 50 % der Längserstreckung des Seitenteils erstreckt. Hierdurch kann gegenüber der in Längsrichtung durchgehenden Verstärkung eine Materialersparnis erzielt werden.

Das Verstärkungsmittel erstreckt sich stets in Querrichtung über den Längsrand des Hauptteils hinaus in Richtung des freien Endes des Seitenabschnitts. Diese Erstreckung des in Richtung des freien Endes des Seitenabschnittes über den Längsrand des Hauptteils hinausgehenden Bereichs des Verstärkungsmittels, gemessen von den seitlichen Längsrändern, beträgt vorzugsweise höchstens 50%, weiter vorzugsweise höchstens 30%, insbesondere höchstens 25%, weiter insbesondere höchstens 20%, weiter insbesondere höchstens 15%, weiter insbesondere höchstens 10% der Quererstreckung des Seitenteils.

Als ganz besonders vorteilhaft erweist es sich, dass ein jeweiliger angefügter Seitenabschnitt rechteckförmig ausgebildet werden kann, ohne dass die erwähnte Einreißproblematik dem entgegenstehen würde.

Das erfindungsgemäß vorgesehene Verstärkungsmittel kann nach einer ersten Ausführungsform der Erfindung in vorteilhafter Weise von einem angefügten Verstärkungsabschnitt gebildet sein, also von einem zusätzlichen dem jeweiligen Seitenabschnitt zugefügten, insbesondere auf den jeweiligen Seitenabschnitt aufgebrachten Material.
Dieser Verstärkungsabschnitt kann beispielsweise streifenförmig ausgebildet sein.
Dieser Verstärkungsabschnitt kann weiterhin jegliche Form annehmen. Dieser Verstärkungsabschnitt kann beispielsweise auch in Form eines Dreiecks ausgebildet sein.

Es kann sich hierbei um einen Abschnitt eines streifen- oder bandförmigen Materials handeln. Insbesondere und vorteilhafterweise kann der Verstärkungsabschnitt von einem Vliesmaterial, einem textilen Material oder einer Folie gebildet sein. Er kann ebenso wie die Seitenabschnitte in einem endlosen Fertigungsprozess im Cut & Place-Verfahren zugeführt und angefügt werden.

Es können auch mehrere Verstärkungsabschnitte vorgesehen sein. Die Verstärkungsmittel können an eine oder beide Oberseiten des Seitenabschnitts angefügt sein.

Der Verstärkungsabschnitt ist vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Als vorteilhaft hat sich ein Polypropylen (PP) Spinnvlies (Spunbond-Material) mit einem Flächengewicht von 20 - 35 g/m², insbesondere von 20 - 30 g/m² erwiesen. Auch Vliesstofflaminate, also insbesondere zweilagige, dreilagige oder mehrlagige Kombinationen können eingesetzt werden. Sollte der Verstärkungsabschnitt aus einer Folie hergestellt werden, wird insbesondere vorgeschlagen diese Folie atmungsaktiv auszubilden.

Das Verstärkungsmittel ist vorteilhafterweise durch Verkleben, thermisches Verschweißen, Ultraschallschweißen, Vernadeln oder Vernähen an eine oder beide Oberseiten des jeweiligen Seitenabschnitts angefügt.

Das Verstärkungsmittel kann dabei mit den vorgenannten Fügeverfahren zumindest abschnittsweise, weiter insbesondere vollflächig an der einen oder an beiden Oberseiten des jeweiligen Seitenabschnittes angebracht sein.

Nach einer besonders vorteilhaften Ausführungsform der Erfindung ist das Verstärkungsmittel von dem Material des jeweiligen Seitenabschnitts selbst gebildet, indem der Seitenabschnitt in dem den Längsrand des Hauptteils überbrückenden Bereich einmal oder mehrmals gefaltet ist. In der Draufsicht auf den eben ausgefalteten Hygieneartikel ist nach dieser Ausführungsform also ein den Längsrand des Hauptteils überfangender oder überlappender Bereich des jeweiligen Seitenabschnitts durch eine Materialverdoppelung oder -vervielfachung durch Falten des Seitenabschnitts gebildet. Hierdurch kann ein besonders effektiver Einreißschutz geschaffen werden. Als besonders vorteilhaft erweist sich eine in der Längsrichtung des Hygieneartikels betrachtete Z-förmige Faltung des jeweiligen Seitenabschnitts.

Die an den Hauptteil angefügten Seitenabschnitte haben im Bereich der Anfügung an den Hauptteil eine Erstreckung in Längsrichtung des Hygieneartikels von vorzugsweise wenigstens 10 cm, insbesondere wenigstens 14 cm, insbesondere wenigstens 18 cm und weiter insbesondere wenigstens 22 cm.

Die Erstreckung eines an den Hauptteil angefügten Seitenabschnitts im entfalteten Zustand in Querrichtung über den Längsrand des Hauptteils hinaus, beträgt wenigstens 5 cm, insbesondere wenigstens 10 cm, insbesondere wenigstens 15 cm, und weiter insbesondere wenigstens 18 cm. Sie beträgt vorzugsweise höchstens 50 cm, vorzugsweise höchstens 35 cm, insbesondere höchstens 30 cm und weiter insbesondere höchstens 27 cm.

Der Überlappungsbereich des jeweiligen Seitenabschnittes mit dem Hauptteil erstreckt sich in Querrichtung vorzugsweise über mindestens 0,5 cm, weiter vorzugsweise über mindestens 1,5 cm, weiter vorzugsweise über mindestens 2,0 cm, weiter insbesondere über mindestens 2,5 cm, weiter insbesondere über höchstens 4,0 cm und weiter insbesondere über höchstens 3,5 cm.

Die Seitenabschnitte sind dabei im Überlappungsbereich vorzugsweise mit den chassisbildenden Materialien des Hauptteils, also insbesondere dem Backsheet und/oder dem Topsheet verbunden.

Vorzugsweise sind die Seitenabschnitte zwischen Backsheet und Topsheet angebracht.

Die an den Hauptteil angefügten Seitenabschnitte sind vorzugsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können Verwendung finden.

Die Verbindung der einzelnen Lagen kann durch an sich übliche und bekannte Verfahren, beispielsweise durch thermische Fügeverfahren (Verschweißung, insbesondere Laserverschweißung, air-through) oder durch Ultraschallschweißverfahren erfolgen; auch die kalte Verpressung, Vernadelung, Vernähung oder Verklebung (beispielsweise mittels Hotmelt) von Vliesstoffmaterialien ist denkbar. Auch die Verbindung mit textilen Geweben, Gewirken oder Gestricken, also mit eine textile Bindung im weitesten Sinne aufweisenden Materialien ist denkbar.

Die an den Hauptteil angefügten Seitenabschnitte können auch aus einer Folie gebildet sein. Sollte vorgesehen sein, den Seitenabschnitt aus einer Folie zu bilden, wird insbesondere vorgeschlagen diese Folie atmungsaktiv auszubilden.

Die an den Hauptteil angefügten Seitenabschnitte können auch als Vlies-Folien-Laminat ausgebildet sein. Sollte vorgesehen sein, den Seitenabschnitt aus einem Vlies-Folien-Laminat zu bilden, so wird insbesondere vorgeschlagen, die dabei integrierte Folie atmungsaktiv auszubilden. Die Verbindung der Lagen kann dabei durch die an sich bekannten Fügeverfahren, wie vorangehend erläutert, erfolgen.

Vorzugsweise werden die seitlich an den Hauptteil angefügten Seitenabschnitte zumindest abschnittsweise atmungsaktiv ausgebildet, wobei insbesondere eine Mikroporosität als vorteilhaft angesehen wird, die sowohl einen Luftaustausch als auch eine Durchlässigkeit für Feuchtigkeit in Form von Wasserdampf gestattet. Vorzugsweise sind sie Seitenabschnitte zudem aus einem für wässrige Flüssigkeiten durchlässigen Material gebildet. Dies beschleunigt den Durchgang von Schweiß von innen nach außen.

Die Seitenabschnitte haben in vorteilhafter Weise ein Flächengewicht von 10 bis 150 g/m², insbesondere 20 - 100 g/m², weiter insbesondere 25 - 50 g/m².

Es erweist sich weiter als vorteilhaft, wenn auf sich selbst gefaltete Bereiche des jeweiligen Seitenabschnitts in dem Überbrückungsbereich des Längsrands des Hauptteils miteinander unlösbar verbunden sind. Solchenfalls wird hierdurch das Verstärkungsmittel gebildet. Wiederum können hierfür an sich beliebige Fügeverfahren und Fügemittel verwendet werden. Der Verwendung von Klebermaterialien wird aufgrund deren zusätzlich verstärkender Wirkung der Vorzug gegeben.
Die Klebermaterialien können dabei vollflächig, streifenförmig, punktuell oder musterförmig aufgetragen sein. Als Klebermaterial wird vorzugsweise ein Hotmelt-Kleber eingesetzt.

Es erweist sich losgelöst von der vorausgehenden Verbesserung des Einreißschutzes angefügter Seitenabschnitte als vorteilhaft, wenn Bereiche der Seitenabschnitte, die in Querrichtung außerhalb des Längsrands liegen, wenigstens um eine in Längsrichtung verlaufende Falzlinie auf sich selbst gefaltet sind. Es sind hierbei in Querrichtung weiter außen liegende Bereiche der Seitenabschnitte gemeint. Hierdurch können die zum Teil weit ausladenden Seitenabschnitte - wie z. B. bei Inkontinenzartikeln - während der Herstellung in schnelllaufenden Prozessen in dieser Konfiguration fixiert werden, so dass sie nicht störend aufflattern. Auch bietet sich dem Benutzer unmittelbar vor der Benutzung des Inkontinenzartikels ein ansprechendes geordnetes Erscheinungsbild.

In Weiterbildung dieses Gedankens erweist es sich als vorteilhaft, wenn die genannten aufeinander gefalteten und flächenhaft aneinander liegenden Teilabschnitte der Seitenabschnitte in dieser gefalteten Konfiguration lösbar entfaltbar aneinander fixiert sind. Auf diese Weise soll also eine für die Zwecke der Herstellung, für den Transport bis kurz vor dem unmittelbaren Gebrauch temporäre lösbare Fixierung erreicht werden.

Es erweist sich insofern auch als vorteilhaft, wenn ein jeweiliger so gefalteter Seitenabschnitt einen Anfassbereich zum Entfalten des Seitenabschnitts aufweist und die lösbare Fixierung beim Entfalten durch einmaliges Ziehen an einem jeweiligen Anfassbereich der Seitenabschnitte auftrennbar ist.

Mit dem erfindungsgemäßen absorbierenden Hygieneartikel wurde ein wesentlich verbesserter Schutz gegen Einreißen der beidseits angefügten Seitenabschnitte an den Hauptteil erreicht. Vorteilhafterweise ist die Einreißfestigkeit im Übergangsbereich von Hauptteil und Seitenabschnitt nach dem nachfolgend zu beschreibenden Test wenigstens 35 N, insbesondere wenigstens 38 N, insbesondere wenigstens 40 N und weiter insbesondere wenigstens 42 N.

Vorteilhafterweise ist die Dehnung bis zum Erreichen der maximalen Zugkraft (Fₘₐₓ) im Zuge des nach dem nachfolgend zu beschreibenden Test wenigstens 110 %, insbesondere wenigstens 113 %, insbesondere wenigstens 115 %, insbesondere wenigstens 118 %, insbesondere wenigstens 120 % und weiter insbesondere wenigstens 122 %.

### Test zur Bestimmung der Einreißfestigkeit:

Die Einreißfestigkeit wird als maximale Kraft bzw. Kraftspitze im Zuge eines Dehnungsversuchs unter Verwendung eines Zugprüfgeräts nach ISO 527-1 (1996) ermittelt. Ein derartiges Zugprüfgerät wird bei Zwick GmbH & Co. KG, Ulm, Deutschland vertrieben. Dabei wird eine zu prüfende Probe in die Klemmen des genannten Zugprüfgeräts eingespannt (Zwick-Klemmen einer Abmessung von 60 mm quer zur Zugrichtung und 30 mm in Zugrichtung). Die Einspannlänge (= Abstand der Klemmen zu Beginn der Zugprüfung) beträgt 45 mm. Es wird eine Vorkraft von 0,2 N angelegt. Dann wird die Messung mit einer Prüfgeschwindigkeit von konstanten 500 mm/min gestartet und die Zugkraft zwischen den Klemmen ermittelt und aufgezeichnet. Die Positionierung der Klemmen an der von einem Inkontinenzartikel gebildeten Probe mit Hauptteil und Seitenabschnitten wird in der Figurenbeschreibung erläutert.

Die Prüfanzahl soll n = 10 betragen. Als Einreißfestigkeit wird bei jeder Einzelmessung die Maximalkraft Fₘₐₓ, also die Kraftspitze innerhalb des aufgezeichneten Kräfteverlaufs ermittelt, und es wird der Mittelwert aus 10 Einzelmessungen dann als Einreißfestigkeit angegeben. Typischerweise bezeichnet die Kraftspitze den Beginn des Einreißens.

Ein weiterer Parameter, der im Zuge der Durchführung des Tests zur Bestimmung der Einreißfestigkeit ermittelt werden kann, ist die Dehnung, gemessen als der Abstand der Klemmen bei Fₘₐₓ in Relation zum Abstand der Klemmen zu Beginn der Zugprüfung (= Einspannlänge), sie wird in Prozent angegeben. Auch hier wird der Mittelwert aus 10 Einzelmessungen zugrundegelegt.

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen des erfindungsgemäßen Inkontinenzartikels. In der Zeichnung zeigt:
- Figur 1: eine Draufsicht auf einen Inkontinenzartikel in schematischer Darstellung mit beidseits angefügten Seitenabschnitten;
- Figuren 2a - e: schematische Darstellungen der Anfügung eines Seitenabschnitts an einen Hauptteil (Figuren b - e mit verschiedenen Verstärkungsmitteln);
- Figur 3: eine Schnittansicht des Anfügungsbereichs eines Seitenabschnitts an den Hauptteil in schematischer Darstellung mit Schnittebene III-III in Figur 2a; und
- Figuren 4 - 6: Figur 3 entsprechende Schnittansichten verschiedener Ausführungsformen der erfindungsgemäßen Anfügung der Seitenabschnitte an den Hauptteil
- Figuren 7 und 8: schematische Schnittansichten eines Inkontinenzartikels mit gefalteten Seitenabschnitten.
- Figuren 9 und 10: schematisch eine Schablone zur Markierung der Anordnung der Klemmen bei der Bestimmung der Einreißfestigkeit
- Figuren 11a und 11b: schematisch die Anordnung des Probenkörpers bei Bestimmung der Einreißfestigkeit

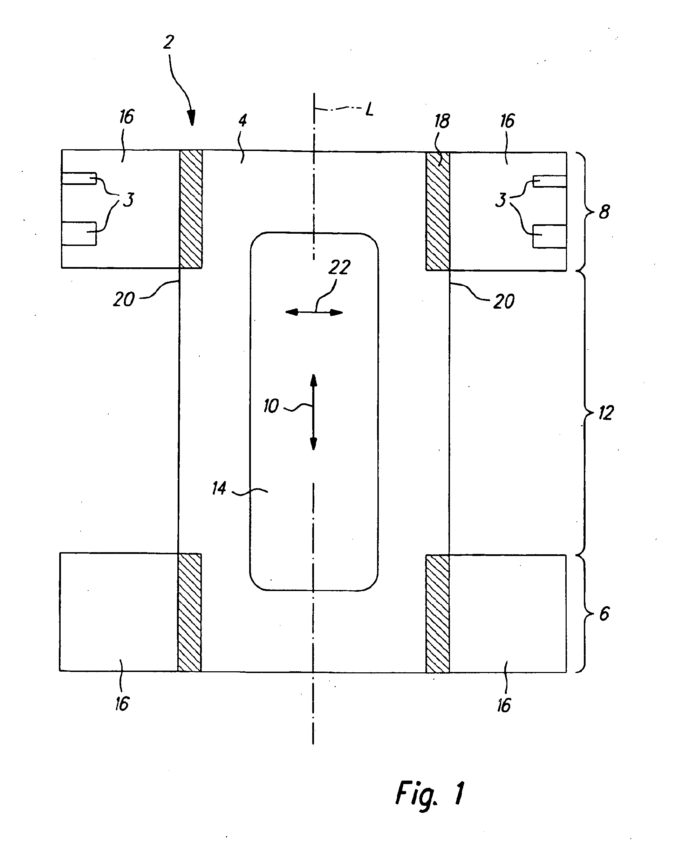

Figur 1 zeigt schematisch eine Draufsicht auf einen absorbierenden Inkontinenzartikel 2 in eben ausgefaltetem Zustand. Der Inkontinenzartikel umfasst einen Hauptteil 4 mit einer Längsmittellinie L bestehend aus einem Vorderbereich 6, einem Rückenbereich 8 und einem in Längsrichtung 10 dazwischen liegenden Schrittbereich 12. Außerdem angedeutet ist ein Saugkörper 14, der üblicherweise zwischen chassisbildenden Materialien des Hauptteils, also insbesondere zwischen einem flüssigkeitsdurchlässigen Topsheet und einem im Wesentlichen flüssigkeitsundurchlässigen Backsheet des Hauptteils 4 angeordnet ist. Es sind aber auch Ausführungsformen denkbar, bei denen der Saugkörper als separate mit Auslaufschutz versehene Einheit auf eine chassisbildende Lage des Hauptteils aufbringbar und dort fixierbar ist.

Der Inkontinenzartikel 2 umfasst des Weiteren Seitenabschnitte 16, die im beispielhaft dargestellten Fall sowohl im Vorderbereich 6 als auch im Rückenbereich 8 als jeweils voneinander separate Materialabschnitte beidseits an den Hauptteil 4 angefügt sind. Sie haben jeweils rechteckförmige Gestalt, was nicht zwingend, jedoch im Hinblick auf die Vermeidung von Schnittabfall vorteilhaft ist. Die Seitenabschnitte sind in einem schraffiert dargestellten Überlappungsbereich 18 mit chassisbildenden Materialien des Hauptteils 4, also beispielsweise mit dem Backsheet und/oder dem Topsheet zum bestimmungsgemäßen Gebrauch unlösbar verbunden. Sie erstrecken sich über seitliche Längsränder 20 des Hauptteils 4 in Querrichtung 22 des Hauptteils 4 bzw. des Inkontinenzartikels 2 hinaus. Die Seitenabschnitte 16 sind dafür gedacht und bestimmt, im angelegten Zustand des Inkontinenzartikels miteinander verbunden zu werden, um einen in Umfangsrichtung durchgehenden Hüftbereich des Hygieneartikels zu bilden. Dabei werden jeweils die auf einer Seite des Hauptteils vorgesehenen Seitenabschnitte miteinander verbunden. Es wäre indessen auch denkbar, dass beispielsweise nur im Rückenbereich 8 Seitenabschnitte 16 vorgesehen sind, die dann auf den Vorderbereich 6 des Hauptteils 4 vermittels der Verschlusselemente 3 festgelegt werden.

Figur 2a zeigt in anderer schematischer Darstellung die Anfügung eines Seitenabschnitts 16 an einen Hauptteil 4 eines Inkontinenzartikels 2. Wiederum ist schraffiert ein Überlappungsbereich 18 von Seitenabschnitt 16 und Hauptteil 4 dargestellt, wie er im Stand der Technik bekannt und üblich ist.

Figuren 2b bis e zeigen jeweils eine erfindungsgemäße Ausbildung des Inkontinenzartikels 2, wobei der schematisch dargestellte Seitenabschnitt 16 ein Verstärkungsmittel 24 aufweist, welches in Querrichtung 22 schmäler ist als der Seitenabschnitt 16. Das Verstärkungsmittel 24 erstreckt sich jedoch in Querrichtung 22 über den Längsrand 20 des Hauptteils 4 hinaus. Das Verstärkungsmittel 24 überfängt dabei den Überlappungsbereich 18 teilweise, wie schematisch in Fig. 2b dargestellt ist. Es erstreckt sich also sowohl über den seitlichen Längsrand 20 in Richtung freies Ende 27 des Seitenabschnitts 16 als auch in Richtung Überlappungsbereich 18, also in Richtung einer Längsmittellinie L des Hauptteils 4.

Das Verstärkungsmittel 24 kann auf verschiedene Weise ausgebildet sein, solange es einen Einreißschutz des Seitenabschnitts 16 bewirkt, insbesondere bei Einleitung einer zur Querrichtung 22 schräg gerichteten Zugkraft auf den Seitenabschnitt 16 bzw. den Überlappungsbereich 18. Das Verstärkungsmittel 24 kann beispielsweise von einem zusätzlichen Verstärkungsabschnitt 26, beispielsweise aus Vlies oder Folie oder aus einem an sich beliebigen verstärkenden Material gebildet sein. Dieses kann durch an sich beliebige Fügeverfahren, insbesondere unter Verwendung eines Klebers, auf das Material des Seitenabschnitts 16 aufgebracht sein.

Bei der Ausführungsform nach Figur 2c überfängt das Verstärkungsmittel 24 den gesamten Überlappungsbereich 18 in der Querrichtung 22.

Bei der Ausführungsform nach Figur 2d erstreckt sich das Verstärkungsmittel 24 in der Längsrichtung 10 von einem dem Schrittbereich zugewandten Querrand 28 des Seitenabschnitts 16 lediglich bis etwa zur Hälfte der Längserstreckung des Seitenabschnitts 16. Dies hat sich für die Bereitstellung eines effektiven Einreißschutzes als hinreichend erwiesen. Die in Figur 2e dargestellte Ausführungsform ist ähnlich der in Figur 2b dargestellten, wobei sich das Verstärkungsmittel 24 wiederum ausgehend vom dem Schrittbereich zugewandten Querrand 28 nur bis etwa zur Hälfte der Längsausdehnung des Seitenabschnitts 16 erstreckt. Bei sämtlichen erfindungsgemäßen Ausführungsformen gemäß Figuren 2b - e wird ein Einreißen ausgehend vom dem Schrittbereich zugewandten Querrand 28 entlang des Längsrandes 20 des Hauptteils 4 verhindert.

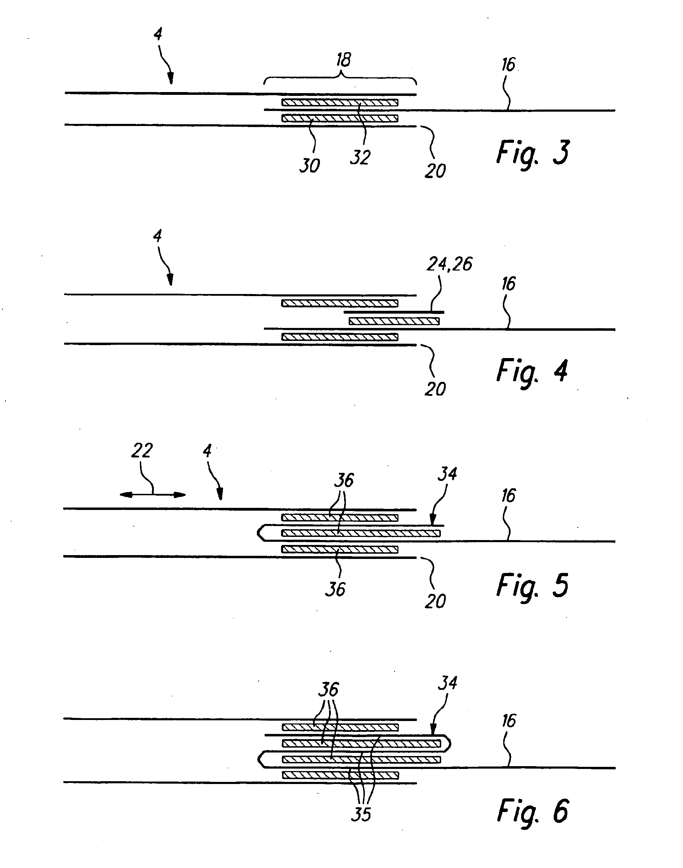

Figur 3 zeigt eine beispielhafte bekannte Ausführungsform der Anfügung eines Seitenabschnitts an einen Hauptteil als Schnittansicht, mit Schnittebene III-III in Figur 2a. In einem Überlappungsbereich 18 erstreckt sich der Seitenabschnitt 16 zwischen zwei Lagen, beispielsweise zwischen Topsheet und Backsheet des Hauptteils 4 eines Hygieneartikels. In diesem Überlappungsbereich 18 ist der Seitenabschnitt 16 mittels einer ersten und einer zweiten Kleberschicht 30, 32 mit den Chassismaterialien unlösbar verbunden. Gerade bei dieser Ausführungsform besteht die Gefahr des Einreißens in der Nähe eines seitlichen Längsrands 20 des Hauptteils 4. Figur 4 zeigt beispielsweise die in Figur 2b angedeutete erfindungsgemäße Ausführungsform, bei der ein Verstärkungsmittel 24 in Form eines Verstärkungsabschnitts 26 derart vorgesehen ist, dass er sich ausgehend von einem Überlappungsbereich 18 über den Längsrand 20 des Hauptteils 4 in der Querrichtung 22 erstreckt.

Figur 5 zeigt eine weitere erfindungsgemäße Ausführungsform, wobei das Verstärkungsmittel 24 von dem Material des Seitenabschnitts 16 selbst gebildet ist, indem der Seitenabschnitt 16 auf sich selbst gefaltet ist, wobei sich die gefaltete Konfiguration 34 in Querrichtung 22 über den Längsrand 20 des Hauptteils 4 hinaus erstreckt. Bei der Ausführungsform gemäß Figur 6 umfasst die gefaltete Konfiguration 34 eine Z-förmige Faltung des Seitenabschnitts 16. Die übereinander gefalteten Bereiche 35 der gefalteten Konfiguration 34 sind jeweils unlösbar aneinander gefügt, beispielsweise mittels flächenhaft oder punktuell aufgetragenen Klebermaterialien 36.

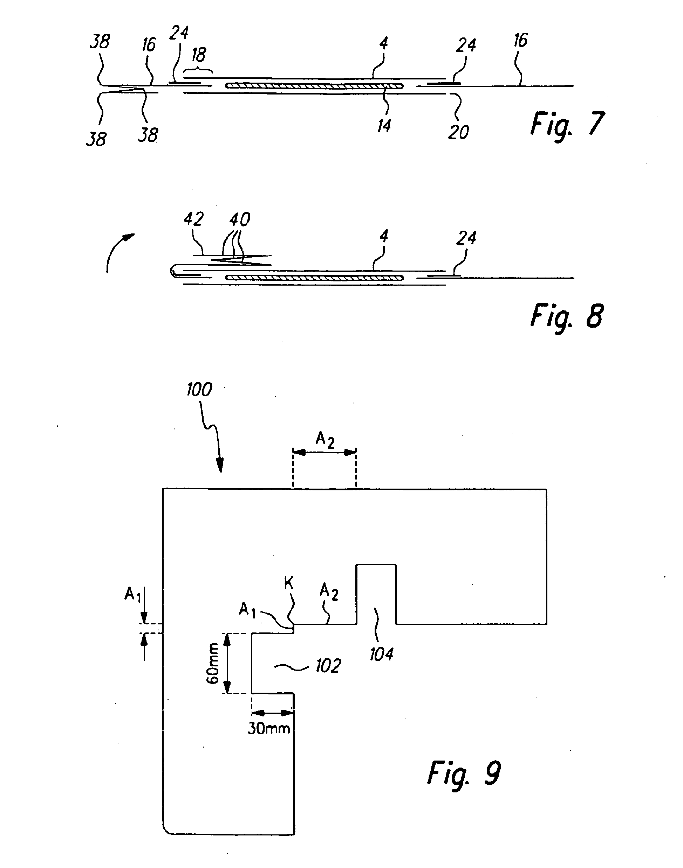

Figuren 7 und 8 zeigen neben dem Verstärkungsmittel 24, welches den Längsrand 20 des Hauptteils 4 des Hygieneartikels überfängt, dass die Seitenabschnitte 16 außerhalb dieses Verstärkungsmittels 24 um mehrere, im dargestellten Fall um drei, in Längsrichtung 10 verlaufende Falzlinien 38 auf sich selbst gefaltet sind. Die so aufeinander gefalteten und flächenhaft aneinander anliegenden Teilabschnitte 40 sind vorzugsweise lösbar entfaltbar aneinander fixiert. Hierfür können beispielsweise Ultraschallschweißpunkte oder sonstige lösbare Fügeverbindungen vorgesehen werden. Durch die lösbare Fixierung der Teilabschnitte 40 aneinander wird während der Handhabung in der schnelllaufenden Herstellungsmaschine ein Aufflattern der Seitenabschnitte 16 verhindert.

Figur 8 zeigt die aufeinandergefaltete Konfiguration der Teilabschnitte 40 in einem nach innen, also auf die Oberseite des Hauptteils 4, gefalteten Zustand. Man erkennt, dass der in Figur 8 oberste Teilabschnitt 40 über die Z-förmig gefaltete Konfiguration nach außen vorsteht und so einen Anfassbereich 42 zum Entfalten des Seitenabschnitts 16 bildet. Vorzugsweise ist die lösbare Fixierung der aufeinandergefalteten Teilabschnitte 40 derart, dass beim Entfalten durch einmaliges Ziehen an dem jeweiligen Anfassbereich 42 in der Querrichtung 22 die Fixierung gelöst und die Seitenabschnitte ganz entfaltbar, also in die in Figur 1 dargestellte Konfiguration, bringbar sind.

### Bestimmung der Einreißfestigkeit

Zur Bestimmung der Einreißfestigkeit nach dem oben beschriebenen Test wird zunächst eine in Figur 9 in der Draufsicht dargestellte Schablone 100 verwendet, deren Ausnehmungen 102, 104 die Anordnung der Klemmen 106, 108 des Zugprüfgeräts beim Einspannen des zu testenden Inkontinenzartikels bezeichnen. Die Abmessung A₁ der Schablone ausgehend von einem Eckpunkt K bis zu der Ausnehmung 102 beträgt 10 mm, und die Abmessung A₂ zu der Ausnehmung 104 beträgt 65 mm. Wie eingangs erwähnt betragen die Abmessungen der Ausnehmungen 102, 104 30 mm und 60 mm und entsprechen den Abmessungen der Klemmen 106, 108 des Zugprüfgeräts.

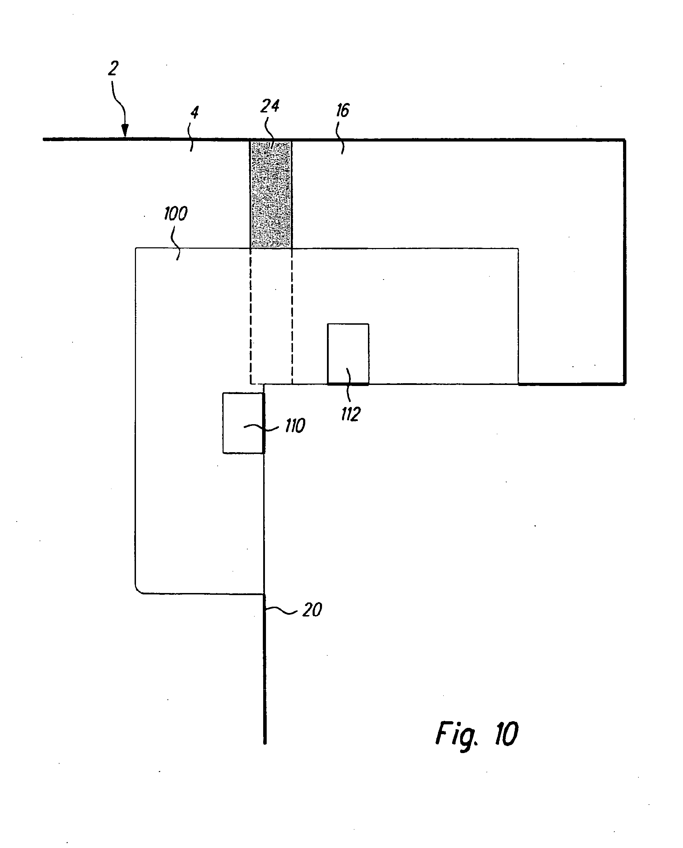

Figur 10 verdeutlicht, wie mit Hilfe der Schablone 100 die Klemmen 106, 108 an dem Inkontinenzartikel 2 befestigt werden. Man erkennt in Figur 10 außerdem das Verstärkungsmittel 24, welches sich im beispielhaft dargestellten Fall in der Längsrichtung 10 über die gesamte Längsausdehnung des Seitenabschnitts 16 im Anfügungsbereich an den Hauptteil 4 erstreckt. Für die Durchführung des Tests ist es wichtig, dass das Verstärkungsmittel 24 nicht von den Klemmen 106, 108 erfasst wird. Dies ist durch Verwendung der Schablone 100 möglich, und es wird außerdem ein Schrägzug auf den Übergangsbereich simuliert. Durch Auflegen der Schablone 100, die bezüglich des Längsrands 20 des Hauptteils 4 und bezüglich des Querrands des Seitenabschnitts 16, wie in Figur 10 dargestellt, orientiert wird, wird der Einspannbereich 110, 112 am Inkontinenzartikel ermittelt, in dem die Klemmen 106, 108 des Zugprüfgeräts positioniert und festgezogen werden.

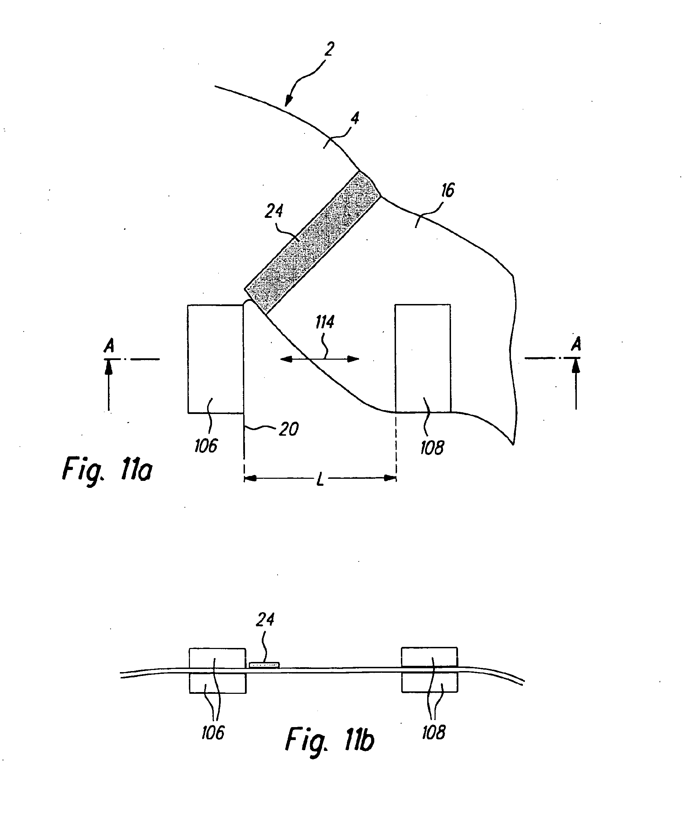

Die Zugprüfung wird dann mit einer Anordnung der Klemmen 106, 108, wie aus Figur 11 ersichtlich, ausgeführt, wobei das Bezugszeichen 114 die Richtung der Bewegung der Klemmen 106, 108 zueinander bezeichnet. Es wird - wie bereits erwähnt - mit einer Einspannlänge L von 45 mm gestartet, wobei die Klemme 106 feststehend ist und die bewegliche Klemme 108 mit einher Prüfgeschwindigkeit von 500 mm/min in der Richtung 114 weggezogen wird. Figur 11b zeigt eine schematische Schnittansicht der Anordnung während der Zugprüfung entsprechend der Schnittebene A-A in Figur 11a.

Die nachfolgenden Tabellen 1 und 2 zeigen jeweils das Ergebnis der Messung eines erfindungsgemäß ausgebildeten Inkontinenzartikels (Spalte "mit Verstärkung") im Vergleich zu einem Inkontinenzartikel ohne Verstärkungsmittel (Spalte "ohne Verstärkung"), jedoch ansonsten identischer Ausbildung. Bei den erfindungsgemäßen Inkontinenzartikeln wurde als Verstärkungsmittel ein Verstärkungsabschnitt aus einem Polypropylen-Spinnvlies mit einem Flächengewicht von 30 g/m² verwendet, das entsprechend der Ausführungsform nach Figur 2c über die gesamte Längsrichtung 10 des Seitenabschnitts und in Querrichtung 22 bis zur inneren Längskante des Seitenabschnitts erstreckt wurde. Der Überstand über den Längsrand 20 des Hauptteils 4 betrug nach innen 2,5 cm und nach außen 1,5 cm.

Die Tabellen 1 und 2 zeigen neben den Kraftspitzen Fₘₐₓ die Dehnung bis Erreichen der Kraftspitze in Prozent bezogen auf die Einspannlänge sowie jeweils den Mittelwert, Standardabweichung und die minimalen und maximalen Messwerte.

Man erkennt, dass die Einreißfestigkeit als Mittelwert von 10 Einzelmessungen mit 41,54 N bzw. als Mittelwert von 10 Einzelmessungen mit 47, 56 N sehr viel höher liegt als bei dem Vergleichsprodukt mit 26,51 N bzw. 31,45 N

Ferner liegt die Dehnung mit 115,08 % bzw. 136,92 % deutlich über der Dehnung von 103,45% bzw. 104,23%.

**Tabelle 1**

| Nr. | F-max [N] **mit Verstärkung** | F-max [N] **ohne Verstärkung** | | Dehnung bis F-max [%] **mit Verstärkung** | Dehnung bis F-max [%] **ohne Verstärkung** |
|---|---|---|---|---|---|
| 1 | 39.59 | 20,07 | | 116,61 | 102,55 |
| 2 | 43,00 | 27,21 | | 118,63 | 95,90 |
| 3 | 41,32 | 33,27 | | 108,47 | 119,18 |
| 4 | 35,14 | 26,98 | | 91,71 | 88,95 |
| 5 | 37.36 | 31,12 | | 115,04 | 97,25 |
| 6 | 34, 89 | 25,82 | | 101,36 | 122,44 |
| 7 | 43,63 | 24,39 | | 119,88 | 96,40 |
| 8 | 49,62 | 20,94 | | 117,33 | 64,96 |
| 9 | 41,30 | 24,62 | | 128,00 | 90,46 |
| 10 | 49,53 | 29,72 | | 133,81 | 156,51 |
| **Mittelwert** | **41,54** | **26,41** | | **115,08** | **103,46** |
| s | 5,19 | 4,20 | | 12,20 | 24.56 |
| min | 34,89 | 20.07 | | 91,71 | 64,96 |
| max | 49,62 | 33, 27 | | 133.81 | 156, 51 |

**Tabelle 2**

| Nr. | F-max [N] **mit Verstärkung** | F-max [N] **ohne Verstärkung** | | Dehnung bis F-max [%] **mit Verstärkung** | Dehnung bis F-max [%] **ohne Verstärkung** |
|---|---|---|---|---|---|
| 1 | 48.17 | 36,38 | | 175,17 | 102,81 |
| 2 | 44,20 | 34,98 | | 122,58 | 122,75 |
| 3 | 46,41 | 31,46 | | 139,51 | 101,73 |
| 4 | 50,58 | 27,02 | | 156,46 | 98,58 |
| 5 | 47,62 | 29,53 | | 140,68 | 99,34 |
| 6 | 43,89 | 36,15 | | 108,45 | 112,07 |
| 7 | 51,64 | 32,12 | | 144,84 | 97,76 |
| 8 | 51,05 | 27,66 | | 149,09 | 103,76 |
| 9 | 48,23 | 32,89 | | 118,85 | 103,75 |
| 10 | 43,84 | 26,33 | | 113,60 | 99, 79 |
| **Mittelwert** | **47,56** | **31,45** | | **136,92** | **104,23** |
| s | 2,96 | 3,73 | | 20,98 | 7,68 |
| min | 43,84 | 26,33 | | 108,45 | 97,76 |
| max | 51,64 | 36,38 | | 175,17 | 122.75 |

## Patentansprüche

1. Absorbierender Inkontinenzartikel (2) mit einem Hauptteil (4), bestehend aus einem Vorderbereich (6), einem Rückenbereich (8) und einem in Längsrichtung (10) dazwischen liegenden zwischen den Beinen eines Benutzers zu liegen kommenden Schrittbereich (12), wobei der Hauptteil (4) einen Saugkörper (14) umfasst, und mit an den Rückenbereich (8) und/oder an den Vorderbereich (6) -beidseits angefügten, voneinander separaten Seitenabschnitten (16), die in einem Überlappungsbereich (18) mit chassisbildenden Komponenten des Hauptteils (4) unlösbar verbunden sind und, sich in Querrichtung (22) über seitliche Längsränder (20) des Hauptteils (4) hinaus erstrecken und den Vorderbereich (6) und den Rückenbereich (8) im angelegten Zustand des Artikels miteinander verbinden, wobei die Seitenabschnitte (16) im Oberlappungsbereich (18) ein Verstärkungsmittel (24)-aufweisen, welches in Querrichtung (22) betrachtet schmäler ausgebildet ist als ein jeweiliger Seitenabschnitt (16) und wobei das Verstärkungsmittel (24) in Längsrichtung im wesentlichen wenigstens nahezu bis zu einem dem Schrittbereich (12) zugewandten Querrand (28) des Seitenabschnitts (16) erstreckt ist oder über den Querrand (28) übersteht, **dadurch gekennzeichnet, dass** die Seitenabschnitte (16) rechteckförmig sind und dass das Verstärkungsmittel (24) in einem den Längsrand (20) des Hauptteils (4) überbrückenden Bereich vorgesehen ist, also ausgehend vom Überlappungsbereich (18) einen seitlichen Längsrandbereich des Hauptteils (4) als auch einen Teil-des Seitenabschnitts (16) in Querrichtung (22) über den Längsrand (20) des Hauptteils (4) hinaus überfängt.

2. Inkontinenzartikel nach Anspruch 1, **dadurch gekennzeichnet, dass** das Verstärkungsmittel (24) in Längsrichtung (10) des Artikels eine geringere Abmessung aufweist als der angefügte Seitenabschnitt (16).

3. Inkontinenzartikel nach einem oder mehreren der vorstehenden Ansprüche, **dadurch gekennzeichnet, dass** das Verstärkungsmittel (24) von einem angefügten Verstärkungsabschnitt (26) gebildet ist.

4. Inkontinenzartikel nach Anspruch 3, **dadurch gekennzeichnet, dass** der Verstärkungsabschnitt (26) streifenförmig ist.

5. Inkontinenzartikel nach Anspruch 3 oder 4, **dadurch gekennzeichnet, dass** der Verstärkungsabschnitt (26) von einem Vliesmaterial, einem textilen Material oder einer Folie gebildet ist.

6. Inkontinenzartikel nach einem oder mehreren der vorstehenden Ansprüche, **dadurch gekennzeichnet, dass** das Verstärkungsmittel (24) durch Verkleben, thermisches Verschweißen, Ultraschallschweißen, Vernadeln oder Vernähen an eine oder beide Oberseiten des Seitenabschnitts (16) angefügt ist.

7. Inkontinenzartikel nach einem oder mehreren der vorstehenden Ansprüche, **dadurch gekennzeichnet, dass** das Verstärkungsmittel (24) von dem Material des Seitenabschnitts (16) selbst gebildet ist, indem der Seitenabschnitt (16) in dem den Längsrand (20) des Hauptteils (4) überbrückenden Bereich einmal oder mehrmals gefaltet ist.

8. Inkontinenzartikel nach Anspruch 7, **dadurch gekennzeichnet, dass** das Verstärkungsmittel (24) von dem Material des Seitenabschnitts (16) selbst gebildet ist und der Seitenabschnitt (16) in dem den Längsrand (20) des Hauptteils (4) überbrückenden Bereich Z-förmig gefaltet ist.

9. Inkontinenzartikel nach Anspruch 7 oder 8, dadurchgekennzeichnet, dass auf sich selbst gefaltete Bereiche (35) des Seitenabschnitts (16) miteinander unlösbar verbunden sind.

10. Inkontinenzartikel nach einem oder mehreren der vorstehenden Ansprüche, **dadurch gekennzeichnet, dass** Bereiche der Seitenabschnitte (16), die in Querrichtung (22) außerhalb des Längsrands (20) liegen, wenigstens um eine in Längsrichtung (10) verlaufende Falzlinie (38) auf sich selbst gefaltet sind.

11. Inkontinenzartikel nach Anspruch 10, **dadurch gekennzeichnet, dass** aufeinander gefaltete und flächenhaft aneinander liegende Teilabschnitte (40) der Seitenabschnitte (16) in dieser gefalteten Konfiguration lösbar entfaltbar aneinander fixiert sind.

12. Inkontinenzartikel nach Anspruch 10 oder 11, **dadurch gekennzeichnet, dass** ein jeweiliger so gefalteter Seitenabschnitt (16) einen Anfassbereich (42) zum Entfalten des Seitenabschnitts (16) aufweist und die lösbare Fixierung beim Entfalten durch einmaliges Ziehen an einem jeweiligen Anfassbereich (42) der Seitenabschnitte (16) auftrennbar ist.

13. Inkontinenzartikel nach einem oder mehreren der vorstehenden Ansprüche, **dadurch gekennzeichnet, dass** die Einreißfestigkeit im Übergangsbereich von Hauptteil und Seitenabschnitt nach dem hier beschriebenen Test wenigstens 35 N, insbesondere wenigstens 38 N, insbesondere wenigstens 40 N und weiter insbesondere wenigstens 42 N beträgt.

14. Inkontinenzartikel nach einem oder mehreren der vorstehenden Ansprüche, **dadurch gekennzeichnet, dass** die Dehnung im Zuge der Durchführung des Tests zur Bestimmung der Einreißfestigkeit bis zum Erreichen der maximalen Zugkraft (Fₘₐₓ) wenigstens 110 %, insbesondere wenigstens 115 %, insbesondere wenigstens, insbesondere wenigstens 118 %, insbesondere wenigstens 120 % und weiter insbesondere wenigstens 122 % beträgt.

## Claims

1. Absorbent incontinence article (2) with a main part (4) composed of a front area (6) and of a rear area (8) and, lying between these in the longitudinal direction (10), a crotch area (12) that comes to lie between the legs of a user, the main part (4) comprising an absorption body (14), and with mutually separate side portions (16) which are joined to the rear area (8) and/or to the front area (6) on both sides wherein the side portions (16) are in an overlapping area (18) in separately joined with chassisforming components of the main part (4) and extend in the transverse direction (22) across lateral longitudinal edges (20) of the main part (4) and connect the front area (6) and the rear area (8) to each other when the article is applied, wherein the side portions (16) have a reinforcing means (24) in the overlapping area (18) which, seen in the transverse direction (22), is designed narrower than a respective side portion (16), wherein the reinforcing means (24) in the longitudinal direction essentially extends at least almost as far as the transverse edge (28) of the side portion (16) facing the crotch area (12) or extends beyond the transverse edge (28), **characterized in that** the side portions (16) are rectangular in shape and that the reinforcing means (24) is provided in an area bridging the longitudinal edge (20) of the main part (4), that is to say starting from the overlapping area (18) in the transverse direction (22) beyond the longitudinal edge (20) of the main part (4) covers both a lateral longitudinal edge area of the main part (4) and also a part of the side portion (16).

2. Incontinence article according to claim 1, **characterized in that** the reinforcing means (24) in the longitudinal direction (10) of the article has a smaller dimension than the attached side portion (16).

3. Incontinence article according to one or more of the previous claims, **characterized in that** the reinforcing means (24) is constituted by an attached reinforcing portion (26).

4. Incontinence article according to the claim 3, **characterized in that** the reinforcing portion (26) is in the shape of a strip.

5. Incontinence article according to the claim 3 or 4, **characterized in that** the reinforcing portion (26) is constituted by a nonwoven material, a textile material, or a foil.

6. Incontinence article according to one or more of the previous claims, **characterized in that** the reinforcing means (24) is attached by gluing, thermal welding, ultrasonic welding, needle-punching, or sewing on one or both topsides of the side portion (16).

7. Incontinence article according to one or more of the previous claims, **characterized in that** the reinforcing means (24) is constituted by the material of the side portion (16) itself, with the side portion (16) being folded once or multiply in the region bridging the longitudinal edge (20) of the main part (4).

8. Incontinence article according to the claim 7, **characterized in that** the reinforcing means (24) is constituted by the material of the side portion (16) itself with the side portion (16) being folded in the shape of a Z in the region bridging the longitudinal edge (20) of the main part (4).

9. Incontinence article according to either one of the claims 7 or 8, **characterized in that** regions (35) of the side portion (16) folded upon themselves are inseparably connected to each other.

10. Incontinence article according to one or more of the previous claims, **characterized in that** regions of the side portions (16) that lie in the transverse direction (22) outside the longitudinal edge (20) are folded upon themselves about at least one fold line (38) extending in the longitudinal direction.

11. Incontinence article according to the claim 10, **characterized in that** partial portions (40) of the side portions (16) folded upon themselves and thus lying against each other over an area are fixed to one another in this folded configuration in such a way that they can be unfolded and separated.

12. Incontinence article according to the claim 10 or 11, **characterized in that** each side portion (16) folded in this manner comprises a grab area (42) for unfolding the side portion (16) and the separable fixture on unfolding is separated by a single pull of the grab area (42) of each of the side portions (16).

13. Incontinence article according to one or more of the previous claims, **characterized in that** the tear resistance in the transitional area between the main part and the side portion is, according to the test described here, at least 35 N, in particular, at least 38 N, in particular, at least 40 N and further in particular, at least 42 N.

14. Incontinence article according to one or more of the previous claims, **characterized in that** the stretch during performance of the test to determine the tear resistance until reaching the maximum tensile force (Fₘₐₓ) is at least 110 %, in particular, at least 115 %, in particular, at least 118 %, in particular, at least 120 % and further in particular, at least 122 %.

## Revendications

1. Article absorbant pour personnes incontinentes (2) comprenant une partie principale (4) composée d'une zone avant (6), d'une zone dorsale (8) et d'une zone d'entrejambe (12) située entre les deux dans le sens longitudinal (10) et venant se placer entre les jambes d'un utilisateur, la partie principale (4) comportant un corps absorbant (14), et comprenant au niveau de la zone dorsale (8) et/ou de la zone avant (6), de chaque côté, des sections latérales (16) séparées l'une de l'autre, lesquelles sections latérales sont reliées de façon inamovible dans une zone de chevauchement (18) avec les composants formant châssis de la partie principale (4) et s'étendent dans le sens transversal (22) au-delà des bords longitudinaux latéraux (20) de la partie principale (4) et relient entre elles la zone avant (6) et la zone dorsale (8) à l'état posé de l'article, les sections latérales (16) présentant dans la zone de chevauchement (18) un moyen de renforcement (24) conçu, vu dans le sens transversal (22), plus étroit qu'une section latérale (16), le moyen de renforcement (24) s'étendant dans le sens longitudinal essentiellement au moins presque jusqu'à un bord transversal (28) de la section latérale (16) orienté vers la zone d'entrejambe (12) ou fait saillie au-dessus du bord transversal (28), **caractérisé en ce que** les sections latérales (16) sont de forme rectangulaire et **en ce que** le moyen de renforcement (24) est prévu dans une zone franchissant le bord longitudinal (20) de la partie principale (4), c'est-à-dire qu'il dépasse, depuis la zone de chevauchement (18), une zone de bord longitudinal latérale de la partie principale (4) ainsi qu'une partie de la section latérale (16) dans le sens transversal (22) et passe au-dessus du bord longitudinal (20) de la partie principale (4).

2. Article pour personnes incontinentes selon la revendication 1, **caractérisé en ce que** le moyen de renforcement (24) présente, dans le sens longitudinal (10) de l'article, des dimensions plus faibles que la section latérale (16) jointe.

3. Article pour personnes incontinentes selon une ou plusieurs des revendications précédentes, **caractérisé en ce que** le moyen de renforcement (24) est formé par une section de renforcement (26) jointe.

4. Article pour personnes incontinentes selon la revendication 3, **caractérisé en ce que** la section de renforcement (26) est en forme de bande.

5. Article pour personnes incontinentes selon la revendication 3 ou 4, **caractérisé en ce que** la section de renforcement (26) est formée par un non-tissé, une matière textile ou par une feuille.

6. Article pour personnes incontinentes selon une ou plusieurs des revendications précédentes, **caractérisé en ce que** le moyen de renforcement (24) est joint à un ou aux deux côtés supérieurs de la section latérale (16) par collage, soudage thermique, soudage par ultrasons, aiguilletage ou par couture.

7. Article pour personnes incontinentes selon une ou plusieurs des revendications précédentes, **caractérisé en ce que** le moyen de renforcement (24) est formé par le matériau de la section latérale (16), la section latérale (16) étant pliée une ou plusieurs fois dans la zone franchissant le bord longitudinal (20) de la partie principale (4).

8. Article pour personnes incontinentes selon la revendication 7, **caractérisé en ce que** le moyen de renforcement (24) est formé par le matériau de la section latérale (16) et **en ce que** la section latérale (16) est pliée en forme de z dans la zone franchissant le bord longitudinal (20) de la partie principale (4).

9. Article pour personnes incontinentes selon la revendication 7 ou 8, **caractérisé en ce que** les zones repliées sur elles-mêmes (35) de la section latérale (16) sont reliées entre elles de façon inamovible.

10. Article pour personnes incontinentes selon une ou plusieurs des revendications précédentes, **caractérisé en ce que** les zones des sections latérales (16), qui se trouvent dans le sens transversal (22) à l'extérieur du bord longitudinal (20), sont pliées sur elles-mêmes au moins autour d'une ligne de pliage (38) s'étendant dans le sens longitudinal (10).

11. Article pour personnes incontinentes selon la revendication 10, **caractérisé en ce que** les sections partielles (40) des sections latérales (16) pliées les unes au-dessus des autres sont fixées entre elles dans cette configuration pliée de façon amovible et dépliable.

12. Article pour personnes incontinentes selon la revendication 10 ou 11, **caractérisé en ce que** chaque section latérale ainsi pliée (16) présente une zone de préhension (42) pour déplier la section latérale (16) et **en ce que** la fixation détachable peut être séparée lors du dépliage en tirant au niveau de la zone de préhension (42) des sections latérales (16).

13. Article pour personnes incontinentes selon une ou plusieurs des revendications précédentes, **caractérisé en ce que** la résistance à la déchirure dans la zone de transition entre la partie principale et la section latérale, conformément au test décrit, est d'au moins 35 N, en particulier d'au moins 38 N, en particulier d'au moins 40 N et plus particulièrement d'au moins 42 N.

14. Article pour personnes incontinentes selon une ou plusieurs des revendications précédentes, **caractérisé en ce que** l'extension pendant la réalisation du test en vue de déterminer la résistance à la déchirure jusqu'à atteindre la force de traction maximale (Fₘₐₓ) est d'au moins 110 %, en particulier d'au moins 115 %, en particulier d'au moins 118%, en particulier d'au moins 120% et plus particulièrement d'au moins 122 %.
